(19) Europäisches Patentamt
European Patent Office
Office européen des brevets

(11) EP 0 558 416 B1

(12) # FASCICULE DE BREVET EUROPEEN

(45) Date de publication et mention de la délivrance du brevet:
**22.05.1996 Bulletin 1996/21**

(51) Int Cl.[6]: **C07J 21/00**, A61K 31/585, C07J 31/00, C07J 41/00

(21) Numéro de dépôt: **93400484.7**

(22) Date de dépôt: **25.02.1993**

(54) **Nouveaux stéroides comportant en position 17 un radical méthylène lactone, leur procédé et des intermédiaires de préparation, leur application comme médicaments et les compositions pharmaceutiques les renfermant**

Neue Steroide mit einer an der 17-Stelle spiro-kondensierte Methylen-Laktongruppe, Verfahren und Zwischenprodukte zu ihrer Herstellung, ihre Verwendung als Arzneimittel und pharmazeutische Präparate davon

New 17-(methylene)-lactone spiro condensed steroids, method and intermediates for their production, their use as medicines and pharmaceutical compositions containing them

(84) Etats contractants désignés:
**AT BE CH DE DK ES FR GB GR IE IT LI LU NL PT SE**

(30) Priorité: **27.02.1992 FR 9202282**

(43) Date de publication de la demande:
**01.09.1993 Bulletin 1993/35**

(73) Titulaire: **ROUSSEL UCLAF**
**F-93230 Romainville (FR)**

(72) Inventeurs:
- **Nedelec, Lucien**
  **F-93340 Le Raincy (FR)**
- **Nique, Francois**
  **F-93320 Pavillons Sous Bois (FR)**
- **Philibert, Daniel**
  **F-94210 La Varenne Saint Hilaire (FR)**

(74) Mandataire: **Vieillefosse, Jean-Claude et al**
**ROUSSEL UCLAF,**
**Département des Brevets,**
**102, Route de Noisy**
**93235 Romainville Cédex (FR)**

(56) Documents cités:
**EP-A- 0 116 974**
**EP-A- 0 190 759**
**EP-A- 0 245 170**

Remarques:
Le dossier contient des informations techniques présentées postérieurement au dépôt de la demande et ne figurant pas dans le présent fascicule.

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 558 416 B1

Printed by Jouve, 75001 PARIS (FR)

## Description

La présente invention concerne de nouveaux stéroïdes comportant en position 17 un radical méthylène lactone, leur procédé et des intermédiaires de préparation, leur application comme médicaments et les compositions pharmaceutiques les renfermant.

Dans les demandes de brevets européens EP-A-245-170 et EP-A-190-759 sont décrites des γ-lactones de l'acide 17β-hydroxy-3-oxo-19-nor-pregna-4,9-dièn-21-carboxylique 11β substitué. Ces produits diffèrent de ceux de la présente demande en ce qu'ils ne comportent pas de groupement méthylène en position alpha de la lactone.

L'invention a pour objet les produits de formule (I) :

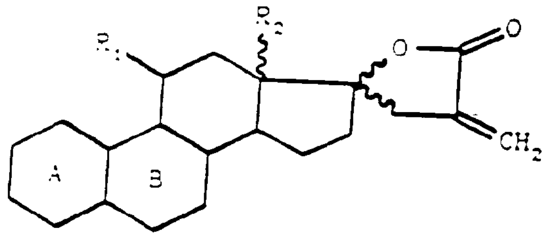

(I)

dans laquelle $R_1$ représente :

soit un radical phényle, biphényle, benzyle ou un radical aryle hétérocyclique choisi parmi les radicaux thiényle, furyle, isothiényle, isofuryle, thiazolyle, isothiazolyle, oxazolyle, isoxazolyle, thiadiazolyle, pyridinyle ou pipéridinyle, chacun de ces radicaux étant éventuellement substitué par un ou plusieurs radicaux choisis parmi :

- les radicaux alkyle, alkényle ou alkynyle ayant au plus 8 atomes de carbone et éventuellement substitués par un ou plusieurs radicaux choisis parmi les radicaux hydroxyle, halogène, oxo, trialkylsilyle, alkoxy ou alkylthio, les radicaux alkyle, alkoxy et alkylthio ayant de 1 à 4 atomes de carbone,
- les radicaux alkoxy, alkényloxy ou alkylthio éventuellement oxydés sous forme de sulfoxyde ou de sulfone, tous ces radicaux ayant au plus 4 atomes de carbone,
- les atomes d'halogène,
- les radicaux trialkylsilyle, chaque radical alkyle comportant de 1 à 4 atomes de carbone,
- les radicaux hydroxyle, trifluorométhyle, acyle ayant de 1 à 6 atomes de carbone, carboxy libre estérifié ou salifié,
- le radical

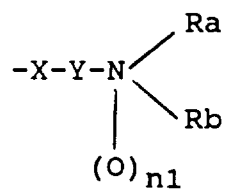

dans lequel X représente une simple liaison, un atome d'oxygène, de soufre ou un radical

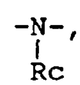

Y représente une simple liaison, un radical alkylène, alkénylène ou alkynylène linéaire ou ramifié ayant au plus 8 atomes de carbone, n1 représente 0 ou 1,

Ra et Rb identiques ou différents représentent :

. un atome d'hydrogène,
. un radical alkyle ayant de 1 à 4 atomes de carbone éventuellement substitué par un radical carboxy libre estérifié ou salifié, un radical trialkylsilyle chaque radical alkyle comportant de 1 à 4 atomes de carbone,
. un radical acyle ayant de 1 à 8 atomes de carbone,
. ou Ra et Rb forment avec l'atome de carbone auquel ils sont liés un hétérocycle comportant éventuellement un autre hétéroatome choisi parmi l'oxygène, le soufre ou l'azote et éventuellement substitué par un radical alkyle ayant de 1 à 4 atomes de carbone, étant entendu que lorsque X représente un atome de soufre, d'oxygène ou un radical

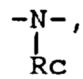

Y ne peut pas être une simple liaison ou un radical méthylène,

Rc représente un atome d'hydrogène ou un radical alkyle ayant de 1 à 4 atomes de carbone,
soit $R_1$ représente un radical indényle ou quinoléinyle éventuellement substitué par un radical alkyle ayant de 1 à 4 atomes de carbone et éventuellement hydrogéné,
$R_2$ en position $\alpha$ ou $\beta$ représente un radical alkyle ayant de 1 à 4 atomes de carbone,
les traits ondulés en position 17 indiquent que le cycle lactonique peut se trouver dans l'une ou l'autre des positions possibles,

les cycles A et B représentent l'une de deux structures suivantes :

a) soit A et B représentent le groupement :

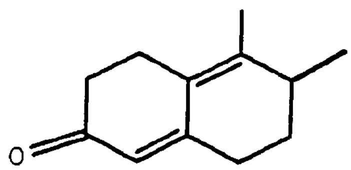

b) soit A et B représentent le groupement :

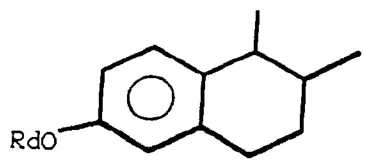

dans lequel Rd représente un atome d'hydrogène ou un radical alkyle ayant de 1 à 4 atomes de carbone éventuellement substitué par un ou plusieurs radicaux choisis parmi les radicaux hydroxyle et les atomes d'halogènes, ou Rd représente un radical arylalkyle ayant au plus 12 atomes de carbone ou un radical acyle ayant de 1 à 6 atomes de carbone,

ainsi que les sels des produits de formule (I) avec les acides et les bases.

Dans ce qui suit, on entend par radical alkyle ayant de 1 à 8 atomes de carbone notamment les radicaux suivants : méthyle, éthyle, propyle isopropyle, butyle, sec-butyle, isobutyle, tert-butyle, pentyle, hexyle, heptyle ou octyle.

- Par radical alkényle ou alkynyle ayant au plus 8 atomes de carbone, on entend notamment les radicaux vinyle, allyle, 1-propényle, éthynyle, propynyle.
- Par radical halogène, on entend les atomes de fluor, chlore, brome ou iode.
- Par radical trialkylsilyle, on entend notamment le triméthylsilyle.
- Par radical alkoxy ayant au plus 4 atomes de carbone, on entend les radicaux méthoxy, éthoxy, propyloxy, isopropyloxy, butyloxy, sec-butyloxy, isobutyloxy ou tert-butyloxy.
- Par radical alkényloxy ayant au plus 4 atomes de carbone, on entend notamment les radicaux vinyloxy, allyloxy ou 1-propényloxy.
- Par radical alkylthio ayant au plus 4 atomes de carbone et éventuellement oxydé sous forme de sulfoxyde ou de sulfone, on entend notamment les radicaux méthylthio, méthylsulfinyle, méthylsulfonyle, éthylthio, éthylsulfinyle, éthylsulfonyle, propylthio, isopropylthio, butylthio, iso-butylthio, secbutylthio, tert-butylthio.
- Par radical acyle ayant de 1 à 8 atomes de carbone, on entend les dérivés d'acides carboxyliques tels que les radicaux formyle, acétyle, propionyle, benzoyle.
- Par radical carboxy estérifié, on entend les esters formés notamment avec les radicaux alkyles ayant au plus 4 atomes de carbone tels que définis ci-dessus et notamment les radicaux éthyle ou tert-butyle.
- Par radical carboxy salifié, on entend les sels de sodium, de potassium, de lithium, de calcium, de magnésium ou d'ammonium. On peut citer, parmi les bases organiques, la méthylamine, la propylamine, la triméthylamine, la diéthylamine, la triéthylamine, la N,N-diméthyléthanolamine, le tris (hydroxyméthyl) amino méthane, l'éthanolamine, la pyridine, la picoline, la dicyclohexylamine, la morpholine, la benzylamine, la procaïne, la lysine, l'arginine, l'histidine, la N-méthylglucamine.
- Par radical alkylène, alkénylène ou alkynylène linéaire ou ramifié, on entend les radicaux divalents formés à partir

des radicaux alkyles, alkényle ou alkynyle tels que par exemple les radicaux méthylène, vinylène ou éthynylène.

- Par radical hétérocyclique que peuvent former Ra et Rb avec l'atome d'azote auquel ils sont liés, on entend notamment les radicaux pipéridinyle, morpholinyle, pipérazinyle, pyrazolidinyle, pyrrolidinyle, imidazolidinyle.

Lorsque $R_1$ comporte une fonction salifiable par un acide et notamment une fonction amino, on obtient des sels d'addition avec les acides.

L'invention s'étend naturellement aux sels d'addition avec les acides des composés de formule (I) salifiables, comme par exemple les sels formés avec les acides chlorhydrique, bromhydrique, nitrique, sulfurique, phosphorique, acétique, formique, propionique, benzoïque, maléique, fumarique, succinique, tartrique, citrique, oxalique, glyoxylique, aspartique, alcane sulfoniques tels que les acides méthane ou éthane sulfoniques, arylsulfoniques, tels que les acides benzène ou paratoluène sulfoniques et arylcarboxyliques.

Parmi les produits de formule (I), on préfère les produits dans lesquels $R_1$ représente soit un radical phényle ou benzyle portant une fonction amine

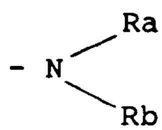

dans laquelle Ra et Rb représentent chacun un radical alkyle primaire, secondaire ou tertiaire renfermant de 1 à 4 atomes de carbone ou Ra et Rb forment avec l'atome d'azote auquel ils sont liés un hétérocycle comportant éventuellement un autre hétéroatome choisi dans le groupe formé par l'oxygène, l'azote et le soufre, soit un radical aryle de préférence phényle portant une fonction méthylthio ou éthylthio, ou une fonction acétyle, ainsi que leurs sels.

Ra et Rb peuvent être identiques ou différents et représenter notamment les valeurs méthyle, éthyle, propyle, isopropyle, butyle, terbutyle, ou Ra et Rb peuvent notamment représenter avec l'atome qui les porte les radicaux morpholinyle, imidazolidinyle, pyrrolidinyle, pipéridinyle, pipérazinyle.

On retient également particulièrement les produits de formule (I) dans laquelle $R_1$ représente un radical phényle et le substituant porté par le radical phényle est en position para, ainsi que leurs sels.

Parmi les valeurs de $R_1$, on préfère les valeurs suivantes :

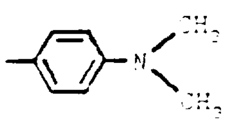 ; 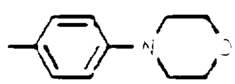 ; 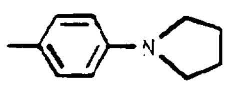 ; 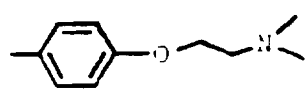

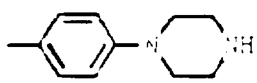 ; 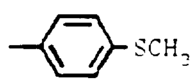 et 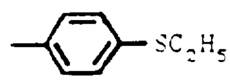

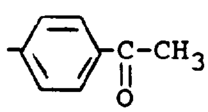

et plus particulièrement encore les valeurs :

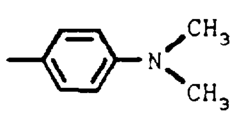 et 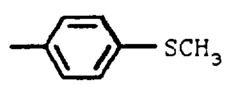

Le radical $R_2$ représente un radical alkyle saturé, linéaire ou ramifié, renfermant de 1 à 4 atomes de carbone, par exemple un radical méthyle, éthyle, propyle ou butyle.

Préférentiellement $R_2$ représente un radical méthyle ou éthyle. Plus préférentiellement $R_2$ représente un radical méthyle.

Le radical $R_2$ peut être en position alpha ou béta. On préfère les produits dans lesquels $R_2$ est en position béta.

De manière générale, on préfère les produits dans lesquels l'atome d'oxygène du cycle méthylène lactone est en position β.

Parmi les produits de formule (I) on préfère les produits répondant à la formule (I') :

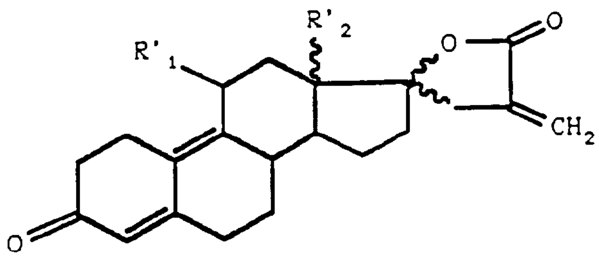

(I')

dans laquelle :

R'$_1$ représente un radical phényle éventuellement substitué par un radical choisi parmi :

- les radicaux alkyle, alkényle, alkynyle, alkoxy ou alkylthio ayant au plus 4 atomes de carbone,
- les radicaux

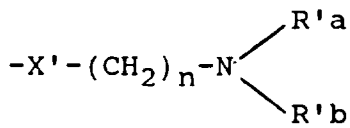

dans lesquels X' représente une simple liaison, un atome d'oxygène ou d'azote,

n représente un entier de 0 à 4, étant entendu que lorsque X' représente un atome d'oxygène ou d'azote, n ne peut pas être égal à 0 ou 1,

R'a et R'b identiques ou différents représentent :

. un atome d'hydrogène,
. un radical alkyle ayant de 1 à 4 atomes de carbone, éventuellement substitué par un radical carboxy libre estérifié ou salifié,
ou
. R'a et R'b forment avec l'atome d'azote auquel ils sont liés un hétérocycle comportant éventuellement un autre hétéroatome choisi parmi l'oxygène et l'azote et éventuellement substitué par un radical alkyle ayant de 1 à 4 atomes de carbone,

R'$_2$ en position α ou β représente un radical méthyle ou éthyle,

les traits ondulés en position 17 indiquent que le cycle lactonique peut se trouver dans l'une ou l'autre des positions possibles.

Plus particulièrement encore, on préfère les produits de formule (I') dans laquelle le substituant R'$_1$ représente un radical phényle éventuellement substitué par un radical dialkylamino ou alkylthio, les radicaux alkyle ayant de 1 à 4 atomes de carbone, et R'$_2$ représente un radical méthyle.

L'invention a spécialement pour objet les produits tels que décrits ci-après dans les exemples et notamment la -γ-lactone de l'acide 17β-hydroxy 11β-[4-(diméthylamino) phényl] 21-méthylène 3-oxo 19-nor 17α-pregna-4,9-diène-21-carboxylique.

L'invention a également pour objet un procédé de préparation des produits de formule générale (I) telle que définie ci-dessus, caractérisé en ce que l'on soumet un produit de formule (II) :

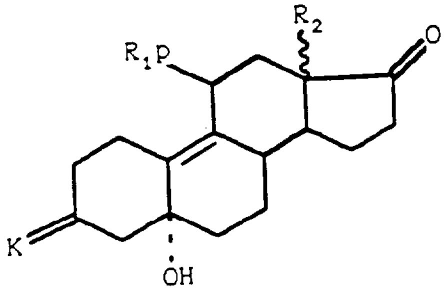

(II)

dans laquelle R$_1$p a les significations indiquées ci-dessus, pour R$_1$ dans lequel les éventuelles fonctions réactives sont éventuellement protégées et K représente une fonction cétonique bloquée, à l'action d'un produit de formule (III) :

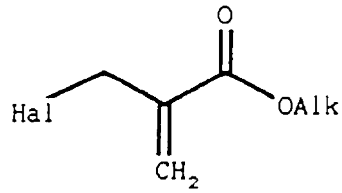

(III)

dans laquelle Hal représente un atome d'halogène et Alk représente un radical alkyle ayant de 1 à 4 atomes de carbone, pour obtenir un produit de formule (IV) :

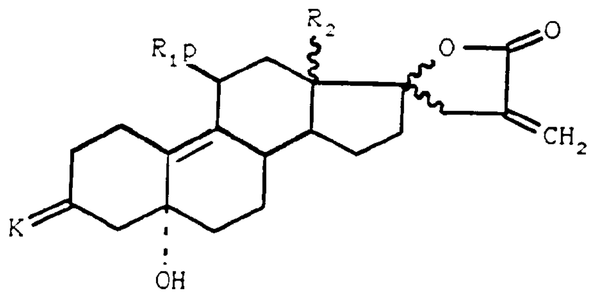

(IV)

sous forme de l'un quelconque des isomères (IVA) ou (IVB) en position 17 ou sous forme d'un mélange des deux isomères, mélange des isomères (IVA) et (IVB) que, si nécessaire ou si désiré, l'on sépare éventuellement en chacun des isomères de formules (IVA) et (IVB) :

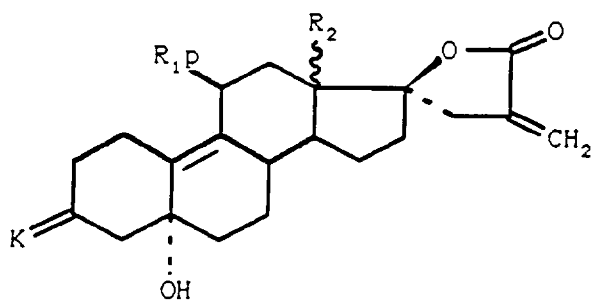

(IVA)

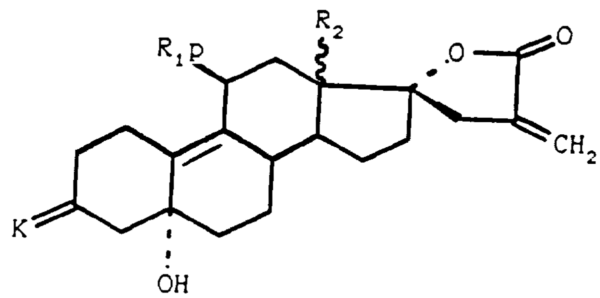

(IVB)

et produits de formules (IVA) et (IVB) sous forme séparée ou sous forme de mélange que l'on soumet à l'action d'un réactif de déshydratation susceptible de libérer la fonction cétone pour obtenir les produits de formules (VA) et (VB) sous forme séparée ou sous forme de mélange :

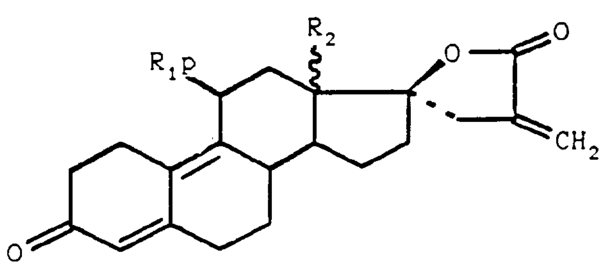

(VA)

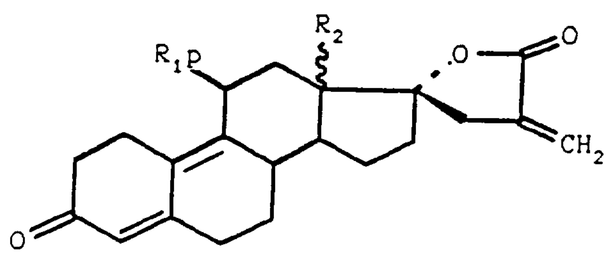

(VB)

et produits de formules (VA) et (VB) que, si nécessaire et si désiré, l'on soumet à l'une ou plusieurs des réactions suivantes, dans un ordre quelconque :

a) séparation du mélange des produits (VA) et (VB) en chacun des constituants (VA) et (VB),
b) élimination du ou des groupements protecteurs que peut porter le substituant $R_1p$,
c) action d'un agent d'aromatisation, puis de saponification puis éventuellement à un réactif d'alkylation ou d'acylation pour obtenir les produits de formule (IB) correspondant aux produits de formule générale (I) dans laquelle les cycles A et B représentent le groupement :

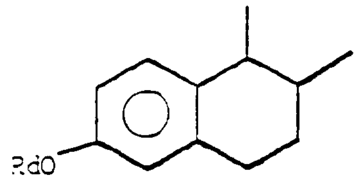

d) salification à l'aide d'un acide ou d'une base des fonctions salifiables que peut comporter le substituant $R_1$.

Dans un mode préférentiel d'exécution du procédé ci-dessus,

1) le radical K représente une fonction cétonique bloquée sous forme de cétal ou de cétal cyclique, tel qu'un diméthyl ou diéthyl cétal éthylène dioxy 2,2-diméthyl propylènedioxy ou thiocétal,
2) les groupements protecteurs des éventuelles fonctions réactives sont choisis parmi les radicaux usuels suivants :

- pour la fonction hydroxyle, les radicaux acyles tels que formyle, acétyle, chloro acétyle, dichloroacétyle, benzoyle, p-nitrobenzoyle, les radicaux éthoxycarbonyle, βββ-trichloroéthoxycarbonyle, benzyloxycarbonyle, tert-butoxycarbonyle, tétrahydropyrannyle, trityle, benzyle,
- pour la fonction amine, les radicaux alkyles tels que tert-butyle, acyles tels que formyle, acétyle, chloroacétyle, propionyle, butyryle, pivaloyle, benzoyle, alkoxycarbonyle tels éthoxycarbonyle, propioxycarbonyle, les radicaux trityle ou trichloroéthyle.

On trouvera une liste de groupements protecteurs utilisables dans la demande de brevet français BF 2.499.995 dont le contenu est incorporé dans la présente demande par référence. 3) l'action des produits de formule (III) sur les produits de formule (II) est effectuée dans un solvant tel que le tétrahydrofuranne, le méthylal ($CH_3$-O-$CH_2$-$OCH_3$) ou le dioxanne. On opère de préférence dans le tétrahydrofuranne.

On utilise de préférence un produit de formule (III) dans laquelle Hal représente un atome de brome ou d'iode, de préférence de brome.

La réaction est conduite de préférence de manière à préparer un organométallique intermédiaire. On préfère utiliser un zincique, on opère alors en présence de zinc. On peut également préparer un cadmien. La réaction peut être accélérée par utilisation d'ultrasons.
4) le mélange des produits de formules IV ou VA et VB est séparé de préférence par chromatographie sur silice.
5) le réactif de déshydratation susceptible de libérer la fonction cétone est de préférence un acide organique ou minéral tel que l'acide chlorhydrique, ou les acides oxalique ou acétique. On peut également utiliser une résine échangeuse d'ions. On peut enfin utiliser un sel acide tel que le sulfate acide de sodium ou de potassium.
6) selon la valeur de $R_1$p, les produits de formules VA et VB constituent ou non des produits de formule (I).

Les produits de formules VA et VB constituent des produits de formule (I) lorsque le groupement $R_1$p ne comporte pas de groupement protecteur dont l'élimination est souhaitée. Dans ce cas, les produits de formules (VA) et (VB) constituent des produits de formule (I) dans laquelle les cycles A et B représentent le groupement :

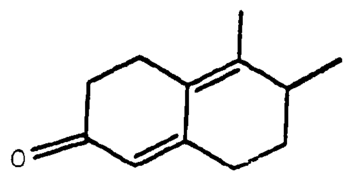

L'élimination des groupements protecteurs que peut porter le substituant $R_1$p est effectuée en général lors de la libération de la fonction cétone.

Il peut cependant se produire, selon la nature des groupements protecteurs utilisés, qu'une ou plusieurs étapes supplémentaires de déprotection soient nécessaires.

On utilise alors les méthodes usuelles telles que l'hydrolyse acide ou basique, l'hydrolyse utilisant l'hydrogène ou la thiourée.

L'hydrolyse acide peut utiliser par exemple un acide choisi dans le groupe formé par les acides chlorhydrique, benzène sulfonique ou paratoluène sulfonique, formique ou trifluoroacétique.

L'hydrolyse basique est effectuée de préférence en présence d'hyddroxyde de sodium ou de potassium. On peut également utiliser la baryte ou un carbonate ou carbonate acide de métal alcalin tel que les carbonates et

carbonates acides de sodium ou de potassium.

On trouvera de même dans le brevet français BF 2.499.995 précité une liste des conditions pouvant être employées et des groupements spécifiques concernés.

7) l'agent d'aromatisation que l'on utilise de préférence un halogénure d'acyle tel que le bromure d'acétyle ou un anhydride d'acide tel que l'anhydride acétique ou un mélange des deux. La saponification subséquente de l'acyle phénolique formé est réalisée dans les conditions usuelles à l'aide d'un agent qui peut être une base alcaline telle que la soude ou la potasse au sein d'un alcool inférieur tel que le méthanol ou l'éthanol.

Le réactif d'alkylation éventuelle des produits phénoliques est un halogénure d'alkyle tel que l'iodure de méthyle. Le réactif d'acylation est un halogénure d'acyle ou un anhydride tels que le chlorure d'acétyle ou l'anhydride acétique.

La salification est effectuée dans des conditions usuelles. On peut opérer, par exemple, en présence de soude éthanolique. On peut également utiliser un sel de sodium tel que le carbonate ou le carbonate acide de sodium ou de potassium.

De même, la salification par un acide est réalisée dans les conditions usuelles. On opère de préférence avec l'acide chlorhydrique, par exemple en solution éthérée.

Les produits de formule (I) ainsi que leurs sels pharmaceutiquement acceptables sont des produits particulièrement intéressants du point de vue pharmacologique.

L'étude des produits sur les récepteurs hormonaux a permis de mettre en évidence des activités glucocorticoïdes ou antiglucocorticoïdes, progestomimétiques ou antiprogestomimétiques, androgènes ou antiandrogènes.

Les produits de formule (I) possèdent en particulier une remarquable activité antiglucocorticoïde.

Les produits de formule (I) possèdent également une activité antiprogestomimétique comme le montrent les résultats des tests exposés ci-après.

Certains produits montrent cependant une activité antiglucocorticoïde supérieure à leur propriété antiprogestomimétique.

Les produits de formule (I) possèdent également une activité antiproliférative qui a été mise en évidence sur différentes lignées tumorales.

Les produits de formule (I) ainsi que leurs sels pharmaceutiquement acceptables qui possèdent des propriétés antiprogestomimétiques peuvent être utilisés comme contraceptifs ; ils peuvent être utilisés contre les dérèglements hormonaux.

Certains produits de formule (I) ainsi que leurs sels pharmaceutiquement acceptables peuvent également présenter des propriétés progestomimétiques et peuvent ainsi être employées dans le traitement des aménorrhées, des dysménorrhées et des insuffisances lutéales.

Les produits de formule (I) ainsi que leurs sels pharmaceutiquement acceptables peuvent donc être utilisés comme médicaments pour lutter principalement contre les effets secondaires des glucocorticoïdes, ils permettent de lutter également contre les troubles dus à une hypersécrétion de glucocorticoïdes et notamment contre le vieillissement en général et plus particulièrement contre l'hypertension, l'athérosclérose, l'ostéoporose, le diabète, l'obésité ainsi que l'immunodépression et l'insomnie.

Les produits de formule (I) ainsi que leurs sels pharmaceutiquement acceptables qui présentent des propriétés antiandrogènes peuvent être utilisés dans le traitement des hypertrophies et du cancer de la prostate, de l'hyperandrogénie, de l'anémie, de l'hirsutisme et de l'acné.

Les propriétés antiprolifératives des produits de formule (I) ainsi que de leurs sels pharmaceutiquement acceptables, les rendent utilisables dans le traitement des cancers hormono-dépendants ou non hormonodépendants, notamment les carcinomes mammaires et leurs métastases ou les cancers du poumon. Ces propriétés les rendent également utilisables dans le traitement des tumeurs bénignes.

Certains des produits de formule (I) ainsi que leurs sels pharmaceutiquement acceptables possèdent des propriétés estrogènes et/ou anti-estrogènes. Les propriétés anti-estrogènes les rendent utilisables dans le traitement des cancers estrogéno dépendants.

Les propriétés estrogènes que peuvent également présenter lesdits produits de formule (I) ainsi que leurs sels pharmaceutiquement acceptables les rendent utilisables également dans le traitement des troubles liés à une hypofolliculinie, par exemple les aménorrhées, les dysménorrhées, les avortement répétés, les troubles prémentruels ainsi que le traitement de la ménopause.

L'invention a donc pour objet à titre de médicament les produits de formule (I) pharmaceutiquement acceptables, c'est-à-dire non toxiques aux doses utilisées, ainsi que leurs sels pharmaceutiquement acceptables.

L'invention a particulièrement pour objet, à titre de médicaments, les produits de formule (I) préférés mentionnés plus haut et tout particulièrement le produit dont le nom suit :

- γ-lactone de l'acide 17β-hydroxy 11β-[4-(diméthylamino) phényl] 21-méthylène 3-oxo 19-nor 17α-pregna-4,9-diène-21-carboxylique.

La posologie utile varie en fonction de l'affection à traiter et de la voie d'administration ; elle peut varier par exemple de 10 mg à 1 g par jour chez l'adulte par voie orale.

Les nouveaux produits de formule (I), et leurs sels, tels que définis ci-dessus peuvent être employés pour préparer des compositions pharmaceutiques renfermant, à titre de principe actif, l'un au moins desdits produits.

Les produits de formule (I) et leurs sels sont utilisés par voie digestive, parentérale ou locale. Ils peuvent être prescrits sous forme de comprimés simples ou dragéifiés, de gélules, de granulés, de suppositoires et de préparation injectables, de pommades, de crèmes, de gels, lesquels sont préparés selon les méthodes usuelles.

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

L'invention a donc pour objet les compositions pharmaceutiques renfermant comme principe actif au moins un produit de formule (I), ou au moins un de leurs sels pharmaceutiquement acceptables.

Les produits de formule (II) sont connus ou peuvent être préparés selon les procédés décrits dans les brevets européens EP 0.057.115, 0.190.759 ou 360.369 ou la demande de brevet international WO 89/12448.

Des exemples de préparation de tels produits sont décrits plus loin dans la partie expérimentale.

Les produits de formule (III) qui sont des dérivés de l'acide méthylacrylique sont des produits connus. C'est ainsi que le 2-(bromométhyl) acrylate d'éthyle est un produit commercial. Une méthode générale de préparation des α-halométhyl acrylates d'éthyle est décrite dans Synthesis Communications, Septembre 1982 p. 924. Les autres produits peuvent être préparés de la même façon.

L'invention a également pour objet, à titre de produits industriels nouveaux, d'une part les produits de formules (IVA) et (IVB), d'autre part les produits de formules (VA) et (VB) dans lesquels le substituant $R_1$p comporte au moins un groupement protecteur.

Les exemples suivants illustrent l'invention sans toutefois la limiter.

**EXEMPLE 1 : γ-lactone de l'acide 17β-hydroxy 11β-[4-(diméthylamino) phényl] 21-méthylène 3-oxo 19-nor 17α-pregna-4,9-diène-21-carboxylique.**

Stade A : γ-lactone de l'acide 5α,17β-dihydroxy 11β-[4-(diméthylamino) phényl] 3,3-(1,2-éthanediyl) bis oxy 21-méthylène 19-nor 17α-pregn-9-ène-21-carboxylique et γ-lactone de l'acide 5α,17α-dihydroxy 11β-[4-(diméthylamino) phényl] 3,3-(1,2-éthanediyl) bis oxy 21-méthylène 19-nor 17β-pregn-9-ène-21-carboxylique.

On introduit sous azote, 1,5 g de zinc en poudre fine et une goutte d'α-bromométhyl acrylate d'éthyle puis ajoute lentement 2,5 ml d'α-bromométhyl acrylate d'étyle en solution dans 5 ml de tétrahydrofuranne sec en maintenant la température à environ 20°C. Après l'addition, on laisse réagir pendant encore 20 minutes à 20°C puis ajoute 2 g de 3,3-[1,2-éthane diyl bis(oxy)] 11β-[(4-diméthylamino) phényl] 5α-hydroxy estr-9-ène-17-one préparé au stade A de l'exemple 7 du brevet européen EP 57.115 en solution dans 8 $cm^3$ de tétrahydrofuranne sec. On laisse réagir 16 heures à température ambiante, verse le mélange réactionnel dans une solution de chlorure d'ammonium et extrait à l'acétate d'éthyle. On lave la phase organique, la sèche et l'évapore. On obtient 3,6 g d'une résine renfermant le mélange des isomères attendus. On chromatographie sur silice (éluant : éther) et obtient 680 mg de la lactone correspondant au produit 17β-hydroxy qui cristallise. On obtient ensuite 550 mg de produit de départ puis enfin 700 mg de la lactone correspondant au produit 17α-hydroxy. On recristallise 1,3 g de ce dernier produit par dissolution dans du chlorure de méthylène, filtration dilution par l'éther, concentration à petit volume, redilution à l'éther et de nouveau concentration de manière à éliminer le chlorure de méthylène. On amène à petit volume, refroidit, essore, lave à l'éther et obtient 1,18 g de produit purifié. F = 232°.

De la même façon, en utilisant le mélange chlorure de méthylène-éther, on recristallise 1,7 g de produit 17β et obtient 1,6 g de produit purifié. F = 205°.

Infra-rouge ($CHCl_3$)

a) Produit 17β-OH

| | |
|---|---|
| C=O | 1755 $cm^{-1}$ |
| C=C | 1665 $cm^{-1}$ |
| OH | 3503 $cm^{-1}$ |

b) Produit 17α-OH

| | |
|---|---|
| C=O | 1752 $cm^{-1}$ |
| C=C | 1666 $cm^{-1}$ |
| OH | 3515 $cm^{-1}$ |

Stade B : γ-lactone de l'acide 17β-hydroxy 11β-[4-(diméthylamino) phényl] 21-méthylène 3-oxo 19-nor 17α-pregna-4,9-diène-21-carboxylique

On met en suspension 0,96 g de γ-lactone de l'acide 5α,17β-dihydroxy 11β-[4-(diméthylamino) phényl] 3,3-(1,2-éthanediyl) bis oxy 21-méthylène 19-nor 17α-pregn-9-ène-21-carboxylique obtenue au stade A dans 15 $cm^3$ de méthanol et ajoute 5 $cm^3$ d'acide chlorhydrique 2N. On abandonne la solution pendant 1 heure à température ambiante, ajoute une solution aqueuse de carbonate acide de sodium et extrait au chlorure de méthylène. La phase organique est lavée à l'eau, séchée, puis évaporée sous pression réduite. On obtient 890 mg de produit attendu. Ce produit est chromatographié sur une colonne de silice (éluant : éther-acétate d'éthyle 9/1). On réunit les éluats, évapore et sèche sous pression réduite. On obtient ainsi 760 mg de produit attendu.

Infra-rouge ($CHCl_3$)

| | |
|---|---|
| diénone | 1655 $cm^{-1}$ |
| | 1612 $cm^{-1}$ |
| aromatique | 1561 $cm^{-1}$ |
| | 1518 $cm^{-1}$ |
| C=O lactone | 1757 $cm^{-1}$ |

**EXEMPLE 2 : γ-lactone de l'acide 17α-hydroxy 11β-[4-(diméthylamino) phényl] 21-méthylène 3-oxo 19-nor 17β-pregna-4,9-diène-21-carboxylique.**

On opère comme au stade B de l'exemple 1 au départ de 1,3 g de γ-lactone de l'acide 5α,17α-dihydroxy 11β-[4-(diméthylamino) phényl] 3,3-(1,2-éthanediyl) bis oxy 21-méthylène 19-nor 17β-pregn-9-ène-21-carboxylique obtenue au stade A de l'exemple 1 et obtient, après purification, 1,03 g de produit attendu. (F = 248°C).

Infra-rouge ($CHCl_3$)

| | |
|---|---|
| diénone | 1655 $cm^{-1}$ |
| aromatique | 1613 $cm^{-1}$ |
| | 1562 $cm^{-1}$ |
| | 1518 $cm^{-1}$ |
| C=O lactone | 1753 $cm^{-1}$ |

**EXEMPLE 3 : γ-lactone de l'acide 17β-hydroxy 11β-[4-(méthylthio) phényl] 21-méthylène 3-oxo 19-nor 17α-pregna-4,9-diène-21-carboxylique.**

Stade A : γ-lactone de l'acide 5α,17β-dihydroxy 11β-[4-(méthylthio) phényl] 3,3-(1,2-éthanediyl) bis oxy 21-méthylène 19-nor 17α-pregn-9-ène-21-carboxylique et γ-lactone de l'acide 5α,17α-dihydroxy 11β-[4-(méthylthio) phényl] 3,3-(1,2-éthanediyl) bis oxy 21-méthylène 19-nor 17β-pregn-9-ène-21-carboxylique.

On opère comme au stade A de l'exemple 1 au départ de 3,18 g de 3,3-[1,2-éthane diyl bis(oxy)] 11β-[(4-méthylthio) phényl] estr-9-ène-5α-hydroxy 17-one préparé comme indiqué dans le brevet européen EP 308.345 et obtient 1,9 g de la lactone du produit 17α-OH et 1,4 g de la lactone du produit 17β-OH.

Infra-rouge ($CHCl_3$)

a) Produit 17β-OH

| | |
|---|---|
| C=O | 1757 $cm^{-1}$ |
| C=C | 1666 $cm^{-1}$ |
| OH | 3510 $cm^{-1}$ |
| aromatiques | 1598 $cm^{-1}$ |
| | 1555 $cm^{-1}$ |
| | 1493 $cm^{-1}$ |

b) Produit 17α-OH

| | |
|---|---|
| C=O | 1754 $cm^{-1}$ |
| C=C | 1667 $cm^{-1}$ |
| | 1627 $cm^{-1}$ |
| OH | 3510 $cm^{-1}$ |
| aromatiques | 1598 $cm^{-1}$ |
| | 1555 $cm^{-1}$ |
| | 1493 $cm^{-1}$ |

Stade B : γ-lactone de l'acide 17β-hydroxy 11β-[4-(méthylthio) phényl] 21-méthylène 3-oxo 19-nor 17α-pregna-4,9-diène-21-carboxylique.

On opère comme au stade B de l'exemple 1 au départ de 1,68 g de γ-lactone de l'acide 5α,17β-dihydroxy 11β-[4-(méthylthio) phényl] 3,3-(1,2-éthanediyl) bis oxy 21-méthylène 19-nor 17α-pregn-9-ène-21-carboxylique obtenue au stade A de l'exemple et obtient, après purification, 825 mg de produit attendu.

Infra-rouge ($CHCl_3$)

| | |
|---|---|
| diénone | 1655 $cm^{-1}$ |
| | 1604 $cm^{-1}$ |
| aromatique | 1556 $cm^{-1}$ |
| | 1493 $cm^{-1}$ |
| C=O lactone | 1759 $cm^{-1}$ |

**EXEMPLE 4 : γ-lactone de l'acide 17α-hydroxy 11β-[4-(méthylthio) phényl] 21-méthylène 3-oxo 19-nor 17β-pregna-4,9-diène-21-carboxylique.**

On opère comme au stade B de l'exemple 1 au départ de 2,35 g de γ-lactone de l'acide 5α,17α-dihydroxy 11β-[4-(méthylthio) phényl] 3,3-(1,2-éthanediyl) bis oxy 21-méthylène 19-nor 17β-pregn-9-ène-21-carboxylique obtenue au stade A de l'exemple 3 et obtient, après purification, 1,18 g de produit attendu.

Infra-rouge ($CHCl_3$)

| | |
|---|---|
| diénone | 1654 $cm^{-1}$ |
| | 1603 $cm^{-1}$ |
| aromatique | 1556 $cm^{-1}$ |
| | 1493 $cm^{-1}$ |
| C=O lactone | 1754 $cm^{-1}$ |

**EXEMPLE 5 : γ-lactone de l'acide 17α-hydroxy 11β-[4-acétylphényl] 21-méthylène 3-oxo 19-nor 17β-pregna-4,9-diène-21-carboxylique et γ-lactone de l'acide 17β-hydroxy 11β-[4-acétylphényl] 21-méthylène3-oxo 19-nor 17α-pregna-4,9-diène-21-carboxylique**

La condensation de l'organozincique est effectuée comme au stade A de l'exemple 1 au départ de 4 g de 3,3-[1,2-éthane diyl bis(oxy)] 11β-[4-(2-méthyl 1,3-dioxolan 2-yl) phényl] estr-9-ène-5α-hydroxy 17-one préparé comme indiqué ci-dessous et obtient 6,9 g d'une résine qui constitue un mélange des lactones des produits 17α-OH et 17β-OH.

L'hydrolyse du mélange est ensuite effectuée comme au stade B de l'exemple 1 au départ des 6,9 g de mélange obtenu ci-dessus et obtient 5,77 g d'un mélange des produits attendus.

Ce mélange est ensuite chromatographié sur une colonne de 250 g de silice (éluant : cyclohexane-acétate d'éthyle 1-1). On obtient ainsi 1,7 g de lactone du produit 17α-OH, 1 g de lactone du produit 17β-OH ainsi que 0,145 g de mélange.

Les 1,7 g de produit 17α-OH sont purifiés une nouvelle fois sur une colonne de 140 g de silice (éluant : cyclohexane-acétate d'éthyle 1-1). On obtient 1,45 g de produit que l'on recristallise dans l'acétate d'éthyle et obtient ainsi 950 mg de cristaux incolores. F = 211°C.

Infra-rouge ($CHCl_3$)

| | |
|---|---|
| diénone | 1680 $cm^{-1}$<br>1659 $cm^{-1}$<br>1604 $cm^{-1}$<br>1567 $cm^{-1}$ |
| C=O lactone | 1755 $cm^{-1}$ |

Les 1 g de produit 17β-OH sont purifiés une nouvelle fois de la même façon sur une colonne de 70 g de silice (éluant : éther-acétate d'éthyle 6-4). On obtient 845 mg de produit que l'on recristallise dans un mélange chlorure de méthylène-éther et obtient ainsi 740 mg de cristaux incolores. F = 196°C.

Infra-rouge ($CHCl_3$)

| | |
|---|---|
| diénone | 1680 $cm^{-1}$<br>1658 $cm^{-1}$<br>1604 $cm^{-1}$<br>1568 $cm^{-1}$ |
| C=O lactone | 1759 $cm^{-1}$ |

**PREPARATION DE L'EXEMPLE 5 : le 3,3-[1,2-éthane diyl bis(oxy)] 11β-[4-(2-méthyl 1,3-dioxolan 2-yl) phényl] estr-9-ène-5α-hydroxy 17-one** utilisé au départ de l'exemple 5 a été préparé comme suit :

On dissout 8,25 g de 3,3-[1,2-éthane diyl bis(oxy)] 5α,10α-époxy estr-9(11)-ène-17-one dans 80 ml de tétrahydrofuranne anhydre et ajoute 825 mg de chlorure cuivreux. On refroidit en agitant sous azote puis à -5°C, ajoute goutte à goutte en 30 minutes, 100 ml d'une solution titrant environ 0,4 mmole/ml du magnésien du 2-(4-bromophényl) 2-méthyl dioxolanne dans le tétrahydrofuranne. L'addition terminée, on agite encore pendant 3 heures à 0°C puis verse le mélange réactionnel dans une solution glacée de chlorure d'ammonium puis extrait à l'acétate d'éthyle.

Après lavage, séchage et évaporation de la phase organique, on obtient 20 g d'une huile que l'on purifie par chromatographie sur silice (éluant : cyclohexane-acétate d'éthyle 1-1) et obtient ainsi 9,8 g de produit attendu.

Infra-rouge ($CHCl_3$)

| | |
|---|---|
| OH en 5 | 3511 $cm^{-1}$ |
| aromatique | 1609 $cm^{-1}$<br>1504 $cm^{-1}$ |
| 17 C=O | 1734 $cm^{-1}$. |

## ETUDE PHARMACOLOGIQUE DES PRODUITS DE L'INVENTION

1 - Etude de l'activité des produits de l'invention sur les récepteurs hormonaux :

**Récepteur progestogène de l'utérus de lapine :**

Des lapines impubères d'environ 1 kg reçoivent une application cutanée de 25 g d'estradiol. 5 jours après ce traitement, les animaux sont sacrifiés, les utérus sont prélevés, pesées et homogénéisés à 0°C, à l'aide d'un Potter teflonverre dans une solution tamponnée TS (Tris 10mM, saccharose 0,25 M, HCl pH 7,4) (1 g de tissu pour 50 ml de TS). L'homogénat est ensuite ultracentrifugé (105 000 g x 90 mn) à 0°C. Des aliquotes du surnageant ainsi obtenu, sont incubées à 0°C pendant un temps t, avec une concentration constante (T) de Produit R tritié (17,21-diméthyl 19-nor 4,9-pregna-diène-3,20-dione) en présence de concentrations croissantes (0 - 2 500. $10^{-9}$M) soit de R froid, soit de progestérone froide, soit du produit froid à tester. La concentration de R tritié lié (B) est ensuite mesurée dans chaque incubat par la technique d'adsorption au charbon dextran.

**Récepteur glucocorticoïde du thymus de rat :**

Des rats mâles Sprague-Dawley EOPS, pesant 160 à 200 g sont surrénalectomisés. 4 à 8 jours après cette ablation, les animaux sont sacrifiés, et les thymus sont prélevés et homogénéisés à 0°C dans un tampon Tris 10mM, saccharose 0,15 M, dithiothreitol 2 mM, HCl pH 7,4 ; à l'aide d'un Potter polytétrafluoroéthylène-verre (1 g de tissu pour 10 ml de TS). L'homogénat est ensuite ultracentrifugé (105 000 g x 90 mn) à 0°C. Des aliquotes du surnageant ainsi obtenu, sont incubées à 0°C pendant un temps (t) avec une concentration constante (T) de dexaméthasone tritiée en présence de concentrations croissantes (0 - 2 500.$10^{-9}$M) soit de dexaméthasone froide, soit du produit froid à tester. La concentration de la dexaméthasone tritiée liée (B) est ensuite mesurée dnas chaque incubat par la technique d'adsorption au charbon-destran.

**Calcul de l'affinité relative de liaison :**

Le calcul de l'affinité relative de liaison (ARL) est identique pour les récepteurs.

On trace les 2 courbes suivantes : le pourcentage de l'hormone tritiée liée $\frac{B}{T}$ en fonction du logarithme de la concentration de l'hormone de référence froide et $\frac{B}{T}$ en fonction du logarithme de la concentration du produit froid testé. On détermine la droite d'équation $I_{50} = (\frac{B}{T}\ max + \frac{B}{T}\ min)/2$ $\frac{B}{T}$ max = Pourcentage de l'hormone tritiée liée pour une incubation de cette hormone tritiée à la concentration (T). $\frac{B}{T}$ min = Pourcentage de l'hormone tritiée liée pour une incubation de cette hormone tritiée à la concentration (T) en présence d'un grand excès d'hormone froide (2 500.$10^{-9}$M).

Les intersections de la droite $I_{50}$ et des courbes permettent d'évaluer les concentrations de l'hormone de référence froide (CH) et du produit froid testé (CX) qui inhibent de 50 % la liaison de l'hormone tritiée sur le récepteur.

L'affinité relative de liaison (ARL) du produit testé est déterminée par l'équation :

$$ARL = 100\ \frac{(CH)}{(CX)}$$

Les résultats obtenus sont les suivants :

| Produits des exemples | Temps d'incubation à 0°C | Progestogène | | Glucocorticoïde | |
|---|---|---|---|---|---|
| | | 2 H | 24 H | 4 H | 24 H |
| 1 | | 32 | 21 | 135 | 94 |
| 2 | | 86 | 36 | 136 | 59 |

2 - Activités antiglucocorticoïde :

La technique utilisée découle de la méthode décrite par Dausse et Coll. dans Molécular Pharmacology 13, 948-955 (1977) ("the relationship beetween glucocorticoïd structure and effects upon Thymocytes"), pour des thymocytes de souris.

Des thymocytes de rats surrénalectomisés sont incubés à 37°C pendant 3 heures, dans un milieu nutritif renfermant 5.$10^{-8}$M de dexaméthasone en présence ou non d'un produit à étudier à différentes concentrations. On ajoute l'uridine tritiée, et poursuit l'incubation pendant une heure. On refroidit les incubats, les traite avec une solution d'acide trichloroacétique à 5 %, les filtre sur papier Whatman GF/A, les lave trois fois à l'aide d'une solution d'acide trifchloroacétique à 5 %. On détermine la radioactivité retenue par le filtre.

Les glucocorticoïdes et en particulier la dexaméthasone provoquent une diminution de l'incorporation d'uridine tritiée. Les produits des exemples 1 à 4 s'opposent à cet effet.

| Produit de l'exemple | $5.10^{-8}$ Dexaméthasone + produit à tester à la concentration de : | % d'inhibition de l'effet de la Dexaméthasone |
|---|---|---|
| 1 | $10^{-8}$M<br>$10^{-7}$M<br>$10^{-6}$M | 71<br>104<br>116 |
| 3 | $10^{-8}$M<br>$10^{-7}$M<br>$10^{-6}$M | 24<br>69<br>133 |

Il a par ailleurs été constaté qu'utilisés seuls les produits testés ne provoquent aucun effet du type glucocorticoïde.

Conclusion des deux tests précédents :

Les produits étudiés présentent une activité antiglucocorticoïde très marquée tout en étant dépourvus d'activité glucocorticoïde.

Par ailleurs, leur relativement faible affinité pour le récepteur progestogène montre une dissociation des activités antiglucocorticoïde et antiprogestomimétique en faveur de la première activité.

## Revendications

**1.** Les produits de formule générale (I) :

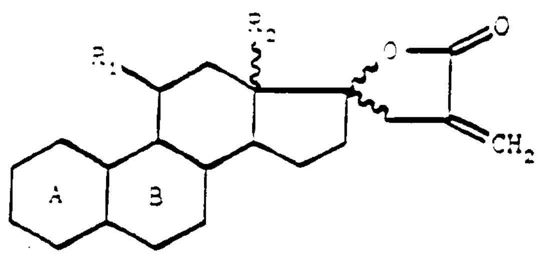

(I)

dans laquelle $R_1$ représente :

soit un radical phényle, biphényle, benzyle ou un radical aryle hétérocyclique choisi parmi les radicaux thiényle, furyle, isothiényle, isofuryle, thiazolyle, isothiazolyle, oxazolyle, isoxazolyle, thiadiazolyle, pyridinyle ou pipéridinyle, chacun de ces radicaux étant éventuellement substitué par un ou plusieurs radicaux choisis parmi :

- les radicaux alkyle, alkényle ou alkynyle ayant au plus 8 atomes de carbone et éventuellement substitués par un ou plusieurs radicaux choisis parmi les radicaux hydroxyle, halogène, oxo, trialkylsilyle, alkoxy ou alkylthio, les radicaux alkyle, alkoxy et alkylthio ayant de 1 à 4 atomes de carbone,
- les radicaux alkoxy, alkényloxy ou alkylthio éventuellement oxydés sous forme de sulfoxyde ou de sulfone, tous ces radicaux ayant au plus 4 atomes de carbone,
- les atomes d'halogène,
- les radicaux trialkylsilyle, chaque radical alkyle comportant de 1 à 4 atomes de carbone,
- les radicaux hydroxyle, trifluorométhyle, acyle ayant de 1 à 6 atomes de carbone, carboxy libre, salifié ou estérifié par un groupement alkyle renfermant au plus 4 atomes de carbone
- le radical

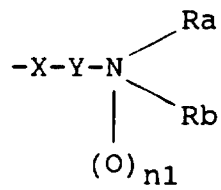

dans lequel X représente une simple liaison, un atome d'oxygène, de soufre ou un radical

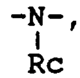

Y représente une simple liaison, un radical alkylène, alkénylène ou alkynylène linéaire ou ramifié ayant au plus 8 atomes de carbone, n1 représente 0 ou 1,

Ra et Rb identiques ou différents représentent :

. un atome d'hydrogène,
. un radical alkyle ayant de 1 à 4 atomes de carbone éventuellement substitué par un radical carboxy libre, estérifié par un groupement alkyle renfermant au plus 4 atomes de carbone ou salifié, un radical trialkylsilyle chaque radical alkyle comportant de 1 à 4 atomes de carbone,
. un radical acyle ayant de 1 à 8 atomes de carbone,
. ou Ra et Rb forment avec l'atome de carbone auquel ils sont liés un hétérocycle comportant éventuellement un autre hétéroatome choisi parmi l'oxygène, le soufre ou l'azote et éventuellement substitué par un radical alkyle ayant de 1 à 4 atomes de carbone, étant entendu que lorsque X représente un atome de soufre, d'oxygène ou un radical

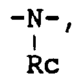

Y ne peut pas être une simple liaison ou un radical méthylène,

Rc représente un atome d'hydrogène ou un radical alkyle ayant de 1 à 4 atomes de carbone,
soit $R_1$ représente un radical indényle ou quinoléinyle éventuellement substitué par un radical alkyle ayant de 1 à 4 atomes de carbone et éventuellement hydrogéné,
$R_2$ en position $\alpha$ ou $\beta$ représente un radical alkyle ayant de 1 à 4 atomes de carbone,
les traits ondulés en position 17 indiquent que le cycle lactonique peut se trouver dans l'une ou l'autre des positions possibles,

les cycles A et B représentent l'une de deux structures suivantes :

a) soit A et B représentent le groupement :

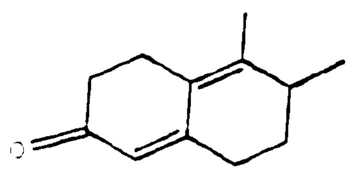

b) soit A et B représentent le groupement :

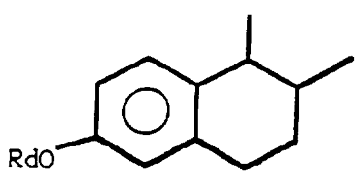

dans lequel Rd représente un atome d'hydrogène ou un radical alkyle ayant de 1 à 4 atomes de carbone éventuellement substitué par un ou plusieurs radicaux choisis parmi les radicaux hydroxyle et les atomes d'halogènes, ou Rd représente un radical arylalkyle ayant au plus 12 atomes de carbone ou un radical acyle ayant de 1 à 6 atomes de carbone,

ainsi que les sels des produits de formule (I) avec les acides et les bases.

**2.** Les produits de formule générale (I) telle que définie à la revendication 1 et répondant à la formule (I') :

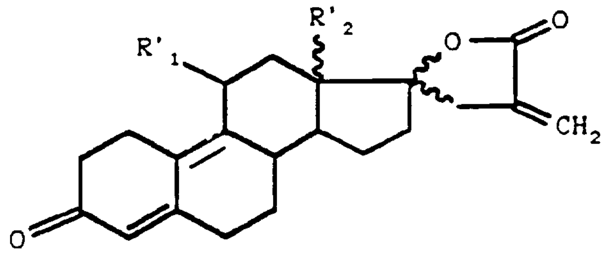

(I')

dans laquelle :

R'$_1$ représente un radical phényle éventuellement substitué par un radical choisi parmi :

- les radicaux alkyle, alkényle, alkynyle, alkoxy ou alkylthio ayant au plus 4 atomes de carbone,
- les radicaux

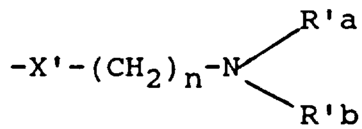

dans lesquels X' représente une simple liaison, un atome d'oxygène ou d'azote,

n représente un entier de 0 à 4, étant entendu que lorsque X' représente un atome d'oxygène ou d'azote, n ne peut pas être égal à 0 ou 1,
R'a et R'b identiques ou différents représentent :

. un atome d'hydrogène,
. un radical alkyle ayant de 1 à 4 atomes de carbone, éventuellement substitué par un radical carboxy libre estérifié ou salifié,
ou
. R'a et R'b forment avec l'atome d'azote auquel ils sont liés un hétérocycle comportant éventuellement un autre hétéroatome choisi parmi l'oxygène et l'azote et éventuellement substitué par un radical alkyle ayant de 1 à 4 atomes de carbone,

R'$_2$ en position $\alpha$ ou $\beta$ représente un radical méthyle ou éthyle,
les traits ondulés en position 17 indiquent que le cycle lactonique peut se trouver dans l'une ou l'autre des positions possibles.

**3.** Les produits de formule (I') telle que définie à la revendication 2 dans laquelle le substituant R'$_1$ représente un radical phényle éventuellement substitué par un radical dialkylamino ou alkylthio, les radicaux alkyle ayant de 1 à 4 atomes de carbone, et R'$_2$ représente un radical méthyle.

**4.** Le produit de formule générale (I) telle que définie à l'une quelconque des revendications 1 à 3 et répondant au nom suivant :

- $\gamma$-lactone de l'acide 17$\beta$-hydroxy 11$\beta$-[4-(diméthylamino) phényl] 21-méthylène 3-oxo 19-nor 17$\alpha$-pregna-4,9-diène-21-carboxylique.

**5.** Procédé de préparation des produits de formule générale (I) telle que définie à la revendication 1 caractérisé en ce que l'on soumet un produit de formule (II) :

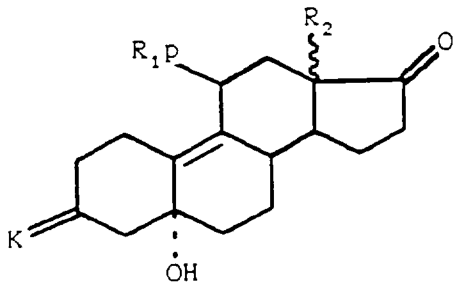

(II)

dans laquelle $R_1p$ a les significations indiquées à la revendication 1 pour $R_1$ dans lequel les éventuelles fonctions réactives sont éventuellement protégées et K représente une fonction cétonique bloquée, à l'action d'un produit de formule (III) :

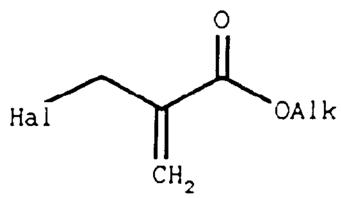

(III)

dans laquelle Hal représente un atome d'halogène et Alk représente un radical alkyle ayant de 1 à 4 atomes de carbone, pour obtenir un produit de formule (IV) :

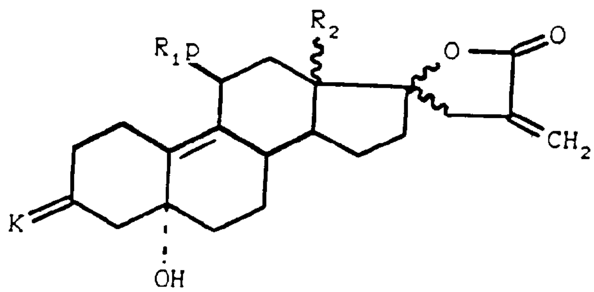

(IV)

sous forme de l'un quelconque des isomères (IVA) ou (IVB) en position 17 ou sous forme d'un mélange des deux isomères, mélange des isomères (IVA) et (IVB) que, si nécessaire ou si désiré, l'on sépare éventuellement en chacun des isomères de formules (IVA) et (IVB) :

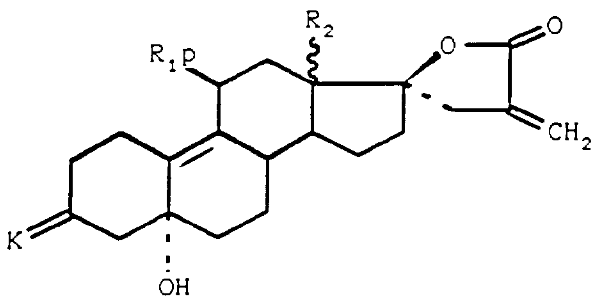

(IVA)

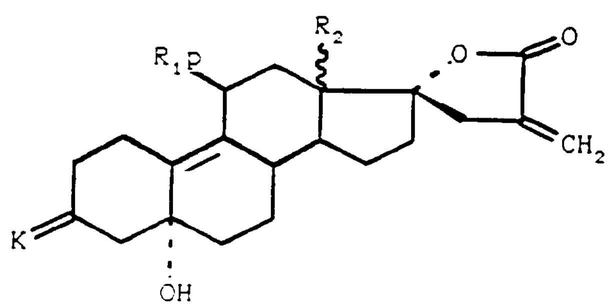

(IVB)

et produits de formules (IVA) et (IVB) sous forme séparée ou sous forme de mélange que l'on soumet à l'action d'un réactif de déshydratation susceptible de libérer la fonction cétone pour obtenir les produits de formules (VA) et (VB) sous forme séparée ou sous forme de mélange :

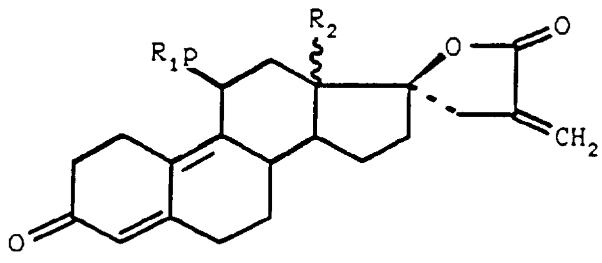

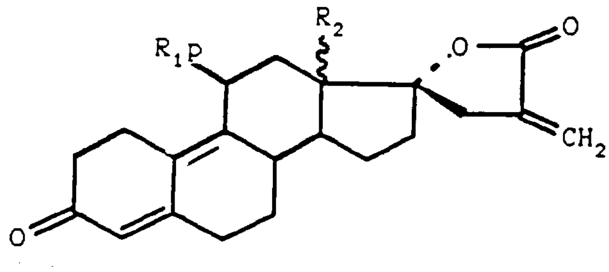

**(VA)** **(VB)**

et produits de formules (VA) et (VB) que, si nécessaire et si désiré, l'on soumet à l'une ou plusieurs des réactions suivantes, dans un ordre quelconque :

a) séparation du mélange des produits (VA) et (VB) en chacun des constituants (VA) et (VB),
b) élimination du ou des groupements protecteurs que peut porter le substituant $R_1p$,
c) action d'un agent d'aromatisation, puis de saponification puis éventuellement à un réactif d'alkylation ou d'acylation pour obtenir les produits de formule (IB) correspondant aux produits de formule générale (I) dans laquelle les cycles A et B représentent le groupement :

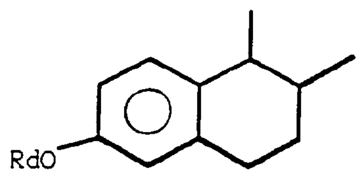

d) salification à l'aide d'un acide ou d'une base des fonctions salifiables que peut comporter le substituant $R_1$.

**6.** A titre de médicaments, les produits de formule générale (I) telle que définie à la revendication 1 pharmaceutiquement acceptables ainsi que leurs sels d'addition avec les acides et les bases pharmaceutiquement acceptables.

**7.** A titre de médicaments, les produits de formule générale (I) telle que définie à l'une quelconque des revendications 2 à 4 pharmaceutiquement acceptables ainsi que leurs sels d'addition avec les acides et les bases pharmaceutiquement acceptables.

**8.** Les compositions pharmaceutiques renfermant, comme principe actif, au moins un médicament défini à l'une des revendications 6 ou 7.

**9.** A titre de produits intermédiaires les produits de formules (IVA), (IVB), (VA) et (VB) définis à la revendication 5, dans lesquels K représente une fonction cétonique bloquée sous forme de cétal cyclique ou non choisi parmi le diméthylcétal, le diéthylcétal, l'éthylène dioxy, le 2,2-diméthylpropylène dioxy ou sous forme du thiocétal correspondant,

dans les produits de formules (VA) et (VB) le substituant $R_1p$ comporte au moins un groupement protecteur,
soit de la fonction hydroxyle choisi parmi les radicaux formyle, acétyle, chloroacétyle, dichloroacétyle, benzoyle, p-nitrobenzoyle, éthoxycarbonyle, βββ-trichloroéthoxycarbonyle, benzyloxycarbonyle, tert-butoxycarbonyle, tétrahydroxypyrannyle, trityle, benzyle,
soit de la fonction amine choisi parmi les radicaux tert-butyle, formyle, acétyle, chloroacétyle, propionyle, butyryle, pivaloyle, benzoyle, éthoxycarbonyle, propioxycarbonyle,
trityle et trichloroéthyle, et dans les produits de formules (IVA) et (IVB) le substituant $R_1p$ représente le substituant $R_1$, tel que défini à la revendication 1, dont les groupements hydroxy ou amino éventuellement présents sont éventuellement protégés comme mentionné ci-dessus,
étant entendu que dans les produits de formules (VA) et (VB) $R_1p$ ne représente pas les mêmes valeurs que $R_1$.

## Patentansprüche

1. Verbindungen der allgemeinen Formel (I) :

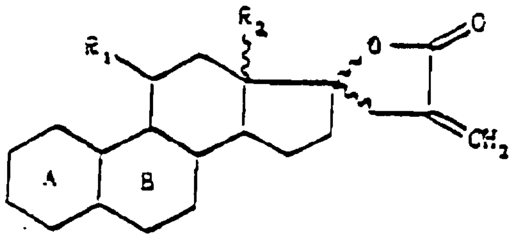

(I)

worin $R_1$,

entweder für einen Phenyl-, Biphenyl-, Benzylrest oder einen heterocyclischen Arylrest, ausgewählt aus Thienyl-, Furyl-, Isothienyl-, Isofuryl-, Thiazolyl-, Isothiazolyl-, Oxazolyl-, Isoxazolyl-, Thiadiazolyl-, Pyridinyl- oder Piperidinylresten, wobei jeder dieser Reste gegebenenfalls durch einen oder mehrere Reste, ausgewählt aus:

- Alkyl-, Alkenyl- oder Alkinylresten mit mindesten 8 Kohlenstoffatomen und gegebenenfalls substituiert durch ein oder mehrere Reste, ausgewählt aus Hydroxyl-, Halogen-, Oxo-, Trialkylsilyl-, Alkoxy- oder Alkylthioresten, wobei die Alkyl-, Alkoxy- und Alkylthioreste 1 bis 4 Kohlenstoffatome besitzen, - Alkoxy- , Alkenyloxy- oder Alkylthioresten, die gegebenenfalls zu einem Sulfoxid oder Sulfon oxidiert sind, wobei alle diese Reste höchstens 4 Kohlenstoffatome besitzen,
- Halogenatomen,
- Trialkylsilylresten, wobei jeder Alkylrest 1 bis 4 Kohlenstoffatome besitzt,
- Hydroxyl-, Trifluormethyl-, Acylresten mit 1 bis 6 Kohlenstoffatomen, einer freien, in ein Salz überführten oder durch einen Alkylrest mit höchstens 4 Kohlenstoffatomen veresterten Carboxylgruppe,
- dem Rest

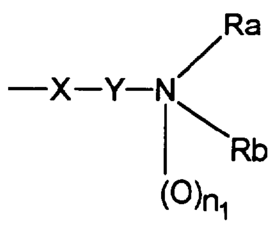

worin X für eine Einfachbindung, ein Sauerstoffatom, ein Schwefelatom oder einen Rest

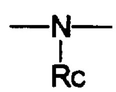

steht, Y für eine Einfachbindung, einen linearen oder verzweigten Alkylen-, Alkenylen- oder Alkinylenrest mit höchstens 8 Kohlenstoffatomen steht, $n_1$ den Wert 0 oder 1 hat,

wobei Ra und Rb, die gleich oder verschieden sind:

. ein Wasserstoffatom,
. einen Alkylrest mit 1 bis 4 Kohlenstoffatomen, der gegebenenfalls durch eine freie Carboxylgruppe substituiert ist, durch einen Alkylrest mit höchstens 4 Kohlenstoffatomen verestert ist, oder in ein Salz überführt ist, einen Trialkylsilylrest, wobei jeder Alkylrest 1 bis 4 Kohlenstoffatome besitzt,
. einen Acylrest mit 1 bis 8 Kohlenstoffatomen, bedeuten,
. oder Ra und Rb mit dem Kohlenstoffatom, an das sie gebunden sind, einen Heterocyclus bilden, der gegebenenfalls ein weiteres Heteroatom, ausgewählt aus Sauerstoff, Schwefel oder Stickstoff, trägt, und gegebenenfalls durch einen Alkylrest mit 1 bis 4 Kohlenstoffatomen substituiert ist, mit der Maßgabe, daß, wenn X für ein Schwefelatom, Sauerstoffatom oder einen Rest

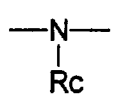

steht, Y keine Einfachbindung oder kein Methylenrest sein kann,

Rc ein Wasserstoffatom oder einen Alkylrest mit 1 bis 4 Kohlenstoffatomen bedeutet, substituiert ist, steht, oder $R_1$ für einen Indenyl- oder Chinoleinylrest, der gegebenenfalls durch eine Alkylrest mit 1 bis 4 Kohlenstoffatomen substituiert ist, und gegebenenfalls hydriert ist, steht,
$R_2$ in Position α oder β einen Alkylrest mit 1 bis 4 Kohlenstoffatomen bedeutet,
die Wellenlinien in Position 17 anzeigen, daß der Lactonring sich in der einen oder der anderen der möglichen Positionen befinden kann,

die Ringe A und B eine der zwei nachstehenden Strukturen bedeuten:

a) entweder bedeuten A und B die Gruppierung

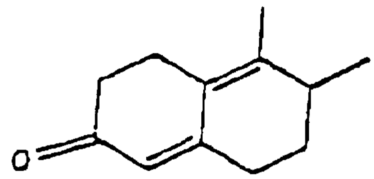

b) oder A und B bedeuten die Gruppierung

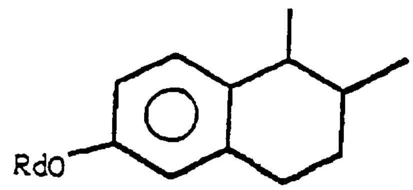

worin Rd ein Wasserstoffatom oder einen Alkylrest mit 1 bis 4 Kohlenstoffatomen, der gegebenenfalls durch einen oder mehrere Rest(e), ausgewählt aus Hydroxylresten und Halogenatomen, substituiert ist, bedeutet, oder Rd einen Arylalkylrest mit höchstens 12 Kohlenstoffatomen oder einen Acylrest mit 1 bis 6 Kohlenstoffatomen bedeutet, sowie die Salze der Verbindungen der Formel (I) mit Säuren und Basen.

**2.** Verbindungen der allgemeinen Formel (I) nach Anspruch 1 entsprechend der Formel (I'):

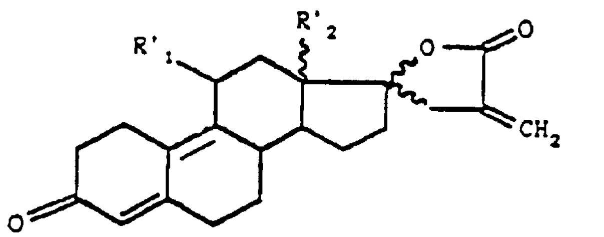

(I')

worin:

$R'_1$ einen Phenylrest bedeutet, gegebenenfalls substituiert durch einen Rest, ausgewählt aus:

- Alkyl-, Alkenyl-, Alkinyl-, Alkoxy- oder Alkylthioresten mit höchstens 4 Kohlenstoffatomen,
- den Resten

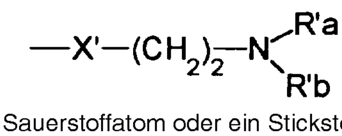

worin X' eine Einfachbindung, ein Sauerstoffatom oder ein Stickstoffatom bedeutet,

n eine ganze Zahl von 0 bis 4 bedeutet, mit der Maßgabe, daß, wenn X' ein Sauerstoff- oder ein Stickstoffatom bedeutet, n nicht den Wert 0 oder 1 haben kann,

R'a und R'b, die gleich oder verschieden sind:

. ein Wasserstoffatom,

. einen Alkylrest mit 1 bis 4 Kohlenstoffatomen, der gegebenenfalls durch eine freie, veresterte oder in ein Salz übergeführte Carboxylgruppe substituiert ist,

bedeuten,

oder

. R'a und R'b mit dem Stickstoffatom, an das sie gebunden sind, einen Heterozyklus, der gegebenenfalls ein weiteres Heteroatom, ausgewählt aus Sauerstoff und Stickstoff, trägt, und gegebenenfalls durch einen Alkylrest mit 1 bis 4 Kohlenstoffatomen substituiert ist, bilden,

$R'_2$ in Position α oder β eine Methyl- oder Ethylgruppe bedeutet,

die Wellenlinien in Position 17 anzeigen, daß der Lactonring sich in der einen oder der anderen der möglichen Positionen befinden kann.

**3.** Verbindungen der Formel (I') nach Anspruch 2, worin der Substituent $R'_1$ einen gegebenenfalls durch einen Dialkylamino- oder Alkylthiorest substituierten Phenylrest bedeutet, wobei die Alkylreste 1 bis 4 Kohlenstoffatome besitzen, und $R'_2$ eine Methylgruppe bedeutet.

**4.** Verbindungen der allgemeinen Formel (I) nach einem der Ansprüche 1 bis 3, nämlich:

- 17β-Hydroxy-11β-[4-(dimethylamino)phenyl]-21-methylen-3-oxo-19-nor-17α-pregna-4,9-dien-21-carbonsäure-γ-lacton.

**5.** Verfahren zur Herstellung der Verbindungen der allgemeinen Formel (I) nach Anspruch 1, dadurch **gekennzeichnet**, daß man eine Verbindung der Formel (II):

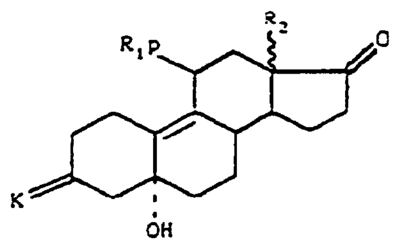

(II)

worin $R_1p$, die in Anspruch 1 für $R_1$ angegebene Definition besitzt, worin die gegebenenfalls vorhandenen reaktiven Funktionen gegebenenfalls geschützt sind, und K eine blockierte Ketonfunktion bedeutet, mit einer Verbindung der Formel (III):

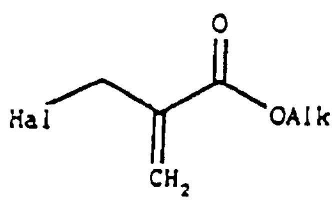

(III)

worin Hal ein Halogenatom bedeutet, und Alk einen Alkylrest mit 1 bis 4 Kohlenstoffatomen bedeutet, umsetzt, wodurch eine Verbindung der Formel (IV) :

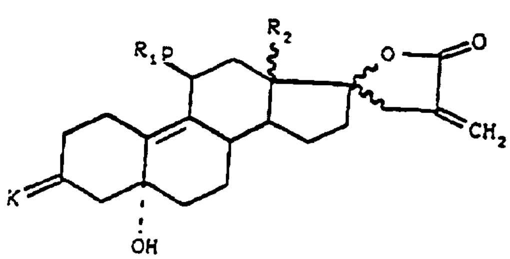

(IV)

in Form eines der Isomeren (IVA) oder (IVB) in Position 17 oder in Form eines Gemisches der zwei Isomeren, eines Gemisches der Isomeren (IVA) und (IVB) erhalten wird, das man gegebenenfalls oder erwünschenfalls gegebenenfalls in jedes der Isomeren der Formel (IVA) und (IVB) aufspaltet:

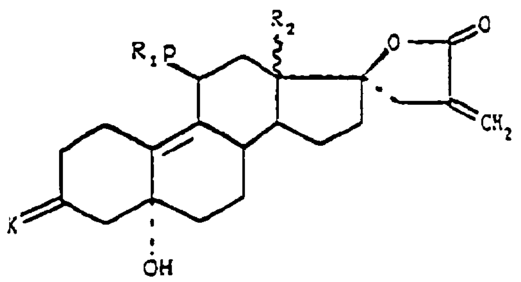

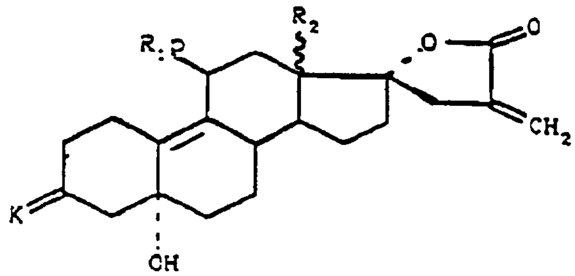

(IVA) (IVB)

und die Verbindungen der Formeln (IVA) und (IVB) in aufgespaltener Form oder in Form des Gemisches mit einem Deshydratisierungsmittel umsetzt, das die Ketonfunktion freisetzen kann, wodurch die Verbindungen der Formeln (VA) und (VB) in getrennter Form oder in Form des Gemisches erhalten werden:

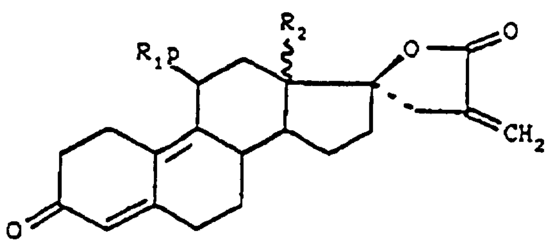

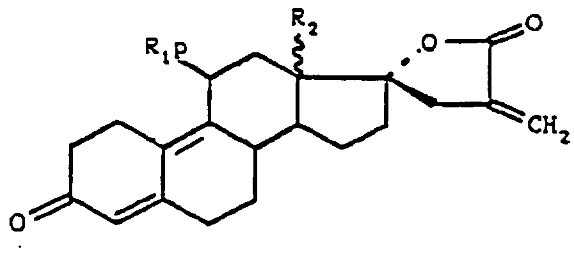

(VA) (VB)

und die Verbindungen der Formeln (VA)und (VB) gegebenenfalls und erwünschtenfalls einer oder mehreren der nachstehenden Reaktionen in irgendeiner Reihenfolge unterwirft:

a) Trennung des Gemisches der Verbindungen (VA)und (VB) in jeden der Bestandteile (VA) und (VB),
b) Abspaltung einer oder der Schutzgruppe(n), die der Substituent $R_1$p tragen kann,
c) Wirkung eines Aromatisierungsmittels, anschließend Verseifung, anschließend gegebenenfalls Umsetzung mit einem Alkylierungs- oder Acylierungsmittel, um die Verbindungen der Formel (IB), die den Verbindungen der allgemeinen Formeln (I), worin die Ringe A und B die Gruppierung:

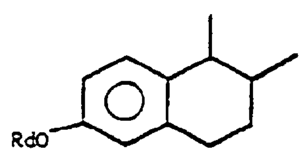

bedeuten, entsprechen, zu erhalten,
d) Salzbildung der in ein Salz überführbaren Funktionen, die der Substituent $R_1$ tragen kann, mit Hilfe einer Säure oder einer Base.

**6.** Pharmazeutisch verträgliche Verbindungen der allgemeinen Formel (I) nach Anspruch 1 sowie ihre Additionssalze mit pharmazeutisch verträglichen Säuren und Basen als Arzneimittel.

**7.** Pharmazeutisch verträgliche Verbindungen der allgemeinen Formel (I) nach einem der Ansprüche 2 bis 4 sowie ihre Additionssalze mit pharmazeutisch verträglichen Säuren und Basen als Arzneimittel.

**8.** Pharmazeutische Zusammensetzungen umfassend als Wirkstoff mindestens ein Arzneimittel nach einem der Ansprüche 6 oder 7.

**9.** Verbindungen der Formeln (IVA), (IVB), (VA) und (VB) nach Anspruch 5 als Zwischenprodukte, worin K eine blokkierte Ketonfunktion in Form des cyclischen oder nichtcyclischen Ketals bedeutet, ausgewählt aus Dimethylketal, Diethylketal, Ethylendioxid, 2,2-Dimethylpropylendioxid oder in Form des entsprechenden Thioketals,

wobei in den Verbindungen der Formeln (VA) und (VB) der Substituent $R_1$p mindestens eine Schutzgruppe trägt,
entweder der Hydroxylfunktion, ausgewählt aus Formyl-, Acetyl-, Chloracetyl-, Dichloracetyl-, Benzoyl-, p-Nitrobenzoyl-, Ethoxycarbonyl-, β,β,β-Trichlorethoxycarbonyl-, Benzyloxycarbonyl-, tert-Butoxycarbonyl-, Tetrahydropyranyl-, Trityl-, Benzylgruppen,
oder der Aminfunktion, ausgewählt aus tert-Butyl-, Formyl-, Acetyl-, Chloracetyl-, Propionyl-, Butyryl-, Pivaloyl-, Benzoyl-, Ethoxycarbonyl-, Propioxycarbonyl-, Trityl- und Trichlorethylgruppen, und wobei in den Verbindungen der Formeln (IVA) und (IVB) der Substituent $R_1$p den Substituenten $R_1$, wie in Anspruch 1 definiert ist, bedeutet, dessen gegebenenfalls vorhandene Hydroxy- oder Aminogruppierungen, gegebenenfalls wie vorstehend erwähnt, geschützt sind,
mit der Maßgabe, daß in den Verbindungen der Formeln (VA) und (VB) $R_1$p nicht die gleichen Bedeutungen wie $R_1$ besitzt.

## Claims

1. The products of general formula (I):

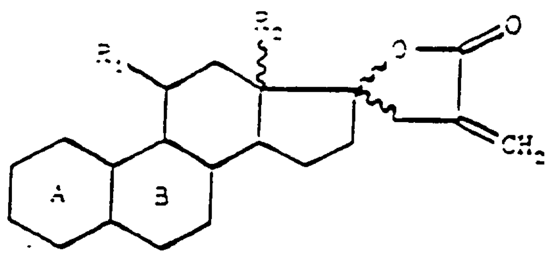

(I)

in which $R_1$ represents:

either a phenyl, biphenyl, benzyl radical or a heterocyclic aryl radical chosen from the following radicals: thienyl, furyl, isothienyl, isofuryl, thiazolyl, isothiazolyl, oxazolyl, isoxazolyl, thiadiazolyl, pyridinyl or piperidinyl, each of these radicals being optionally substituted by one or more radicals chosen from:

- alkyl, alkenyl or alkynyl radicals having at most 8 carbon atoms and optionally substituted by one or more radicals chosen from hydroxyl, halogen, oxo, trialkylsilyl, alkoxy or alkylthio radicals, the alkyl, alkoxy and alkylthio radicals having 1 to 4 carbon atoms,
- alkoxy, alkenyloxy or alkylthio radicals optionally oxidized in the form of the sulphoxide or sulphone, all these radicals having at most 4 carbon atoms,
- halogen atoms,
- trialkylsilyl radicals, each alkyl radical containing 1 to 4 carbon atoms,
- hydroxyl, trifluoromethyl, acyl having 1 to 6 carbon atoms, carboxy radicals free, salified, esterified by an alkyl group containing at most 4 carbon atoms,
- the radical

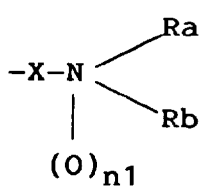

in which X represents a single bond, an oxygen or sulphur atom or an

$$-\underset{\displaystyle Rc}{\underset{|}{N}}-$$

radical, Y represents a single bond, a linear or branched alkylene, alkenylene or alkynylene radical having at most 8 carbon atoms, n1 represents 0 or 1,

Ra and Rb, being identical or different, represent:

- . a hydrogen atom,
- . an alkyl radical having 1 to 4 carbon atoms optionally substituted by a carboxy radical free, esterified by an alkyl group containing at most 4 carbon atoms or salified, a trialkylsilyl radical, each alkyl radical containing 1 to 4 carbon atoms,
- . an acyl radical having 1 to 8 carbon atoms,

or Ra and Rb form with the carbon atom to which they are linked a heterocycle optionally containing another heteroatom chosen from oxygen, sulphur or nitrogen and optionally substituted by an alkyl radical having 1 to 4 carbon atoms, it being understood that when X represents a sulphur or oxygen atom or an

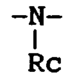

radical, Y cannot be a single bond or a methylene radical,

Rc represents a hydrogen atom or an alkyl radical having 1 to 4 carbon atoms,
<u>or</u> $R_1$ represents an indenyl or quinolinyl radical optionally substituted by an alkyl radical having 1 to 4 carbon atoms and optionally hydrogenated,
$R_2$ in alpha or beta position represents an alkyl radical having 1 to 4 carbon atoms,
the wavy lines in position 17 indicate that the lactone ring can be found in one or other of the possible positions, as well as the salts of the products of formula (I) with acids and bases.

the rings A and B represent one of the two following structures:

a) either A and B represent the group:

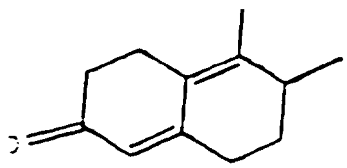

b) or A and B represent the group:

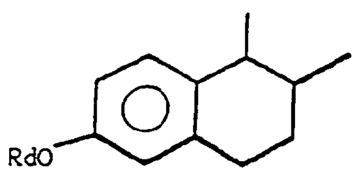

in which Rd represents a hydrogen atom or an alkyl radical having 1 to 4 carbon atoms optionally substituted by one or more radicals chosen from hydroxyl radicals and halogen atoms, or Rd represents an arylalkyl radical having at most 12 carbon atoms or an acyl radical having 1 to 6 carbon atoms,

**2.** The products of general formula (I) as defined in claim 1 and corresponding to the formula (I'):

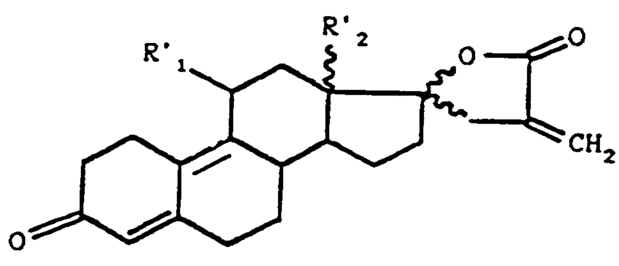

(I')

in which:

R'$_1$ represents a phenyl radical optionally substituted by a radical chosen from:

- alkyl, alkenyl, alkynyl, alkoxy or alkylthio radicals having at most 4 carbon atoms,

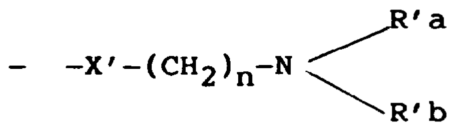

radicals
in which X' represents a single bond, an oxygen or nitrogen atom,

n represents an integer from 0 to 4, it being understood that when X' represents an oxygen or nitrogen atom, n cannot be equal to 0 or 1,

R'a and R'B, identical or different, represent:

. a hydrogen atom,
. an alkyl radical having 1 to 4 carbon atoms, optionally substituted by a free, esterified or salified carboxy radical, or
. R'a and R'b form with the nitrogen atom to which they are linked a heterocycle optionally containing another heteroatom chosen from oxygen and nitrogen and optionally substituted by an alkyl radical having 1 to 4 carbon atoms, $R'_2$ in alpha or beta position represents a methyl or ethyl radical,

the wavy lines in position 17 indicate that the lactone ring can be found in one or other of the possible positions.

**3.** The products of formula (I') as defined in claim 2 in which the $R'_1$ substituent represents a phenyl radical optionally substituted by a dialkylamino or alkylthio radical, the alkyl radicals having 1 to 4 carbon atoms, and $R'_2$ represents a methyl radical.

**4.** The product of general formula (I) as defined in any one of claims 1 to 3 and corresponding to the following name:

- γ-lactone of 17beta-hydroxy 11beta-[4-(dimethylamino) phenyl] 21-methylene 3-oxo 19-nor 17alpha-pregna-4,9-diene-21-carboxylic acid.

**5.** Preparation process for the products of general formula (I) as defined in claim 1, characterized in that a product of formula (II):

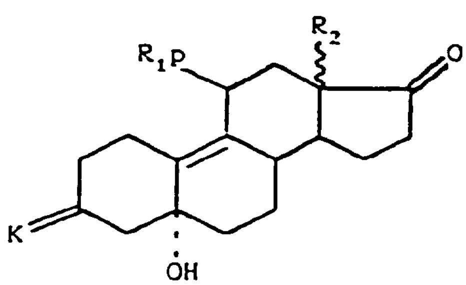

(II)

in which $R_1p$ has the meanings indicated in claim 1 for $R_1$ in which the optional reactive functions are optionally protected and K represents a blocked ketone function, is subjected to the action of a product of formula (III):

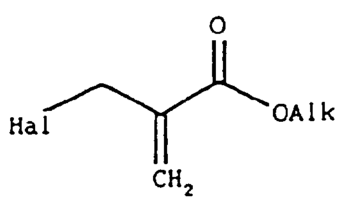

(III)

in which Hal represents a halogen atom and Alk represents an alkyl radical having 1 to 4 carbon atoms, in order to obtain a product of formula (IV):

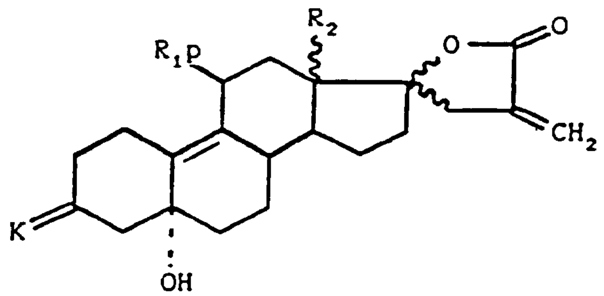

(IV)

in the form of any one of the (IVA) or (IVB) isomers in position 17 or in the form of a mixture of two isomers, which mixture of (IVA) and (IVB) isomers, if necessary or if desired, is optionally separated into each of the isomers of formulae (IVA) and (IVB):

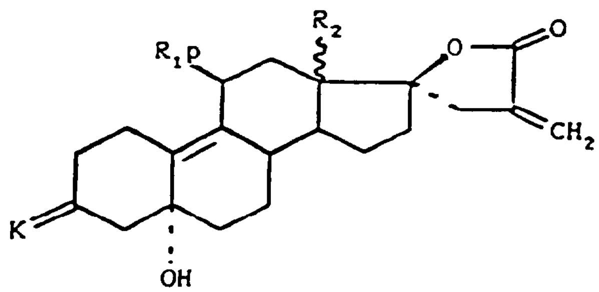

(IVA)

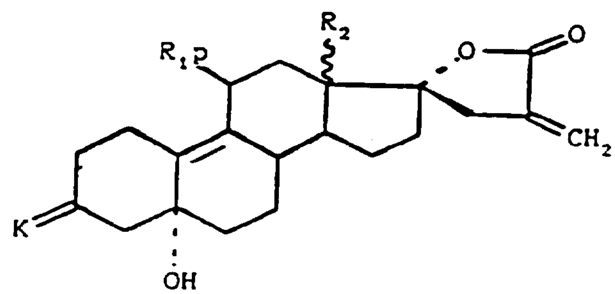

(IVB)

which products of formulae (IVA) and (IVB) in a separated form or in the form of a mixture are subjected to the action of a dehydration reagent capable of releasing the ketone function in order to obtain the products of formulae (VA) and (VB) in a separated form or in the form of a mixture:

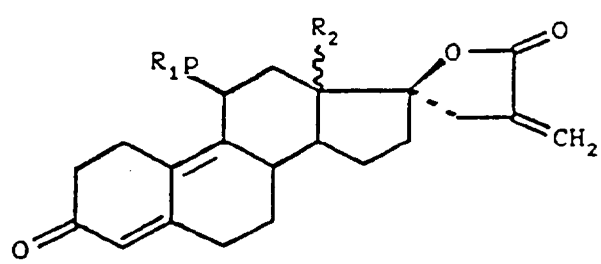

(VA)

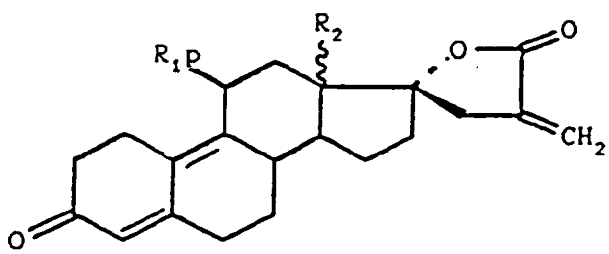

(VB)

and which products of formulae (VA) and (VB), if necessary and if desired, are subjected to one or more of the following reactions, in any order:

a) separation of the mixture of products (VA) and (VB) into each of the (VA) and (VB) constituents,
b) elimination of the protective group or groups which can be carried by the $R_1p$ substituent,
c) the action of an aromatization agent, then of a saponification agent then optionally to an alkylation or acylation reagent in order to obtain the products of formula (IB) corresponding to the products of general formula (I) in which rings A and B represent the group:

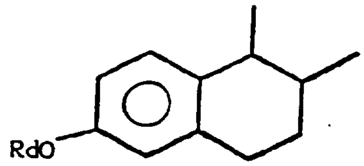

d) salification using an acid or a base of the salifiable functions that can be contained by the $R_1$ substituent.

**6.** As medicaments, the pharmaceutically acceptable products of general formula (I) as defined in claim 1 as well as their addition salts with pharmaceutically acceptable acids and bases.

**7.** As medicaments, the pharmaceutically acceptable products of general formula (I) as defined in any one of claims 2 to 4 as well as their addition salts with pharmaceutically acceptable acids and bases.

**8.** The pharmaceutical compositions containing, as active ingredient, at least one medicament defined in one of claims 6 or 7.

**9.** As intermediate products, the products of formulae (IVA), (IVB), (VA) and (VB) defined in claim 5, in which K represents a ketone function blocked in the form of a cyclic or non-cyclic ketal chosen from dimethylketal, diethylketal, ethylene dioxy, 2,2-dimethylpropylene dioxy or in the form of the corresponding thioketal,

in the products of formulae (VA) and (VB) the substituent $R_1p$ contains at least one protective group,
either of the hydroxyl function chosen from the following radicals: formyl, acetyl, chloroacetyl, dichloroacetyl, benzoyl, p-nitrobenzoyl, ethoxycarbonyl, beta,beta,betatrichloroethoxycarbonyl, benzyloxycarbonyl, tert-butoxycarbonyl, tetrahydroxypyrannyl, trityl, benzyl,
or of the amine function chosen from the following radicals: tert-butyl, formyl, acetyl, chloroacetyl, propionyl, butyryl, pivaloyl, benzoyl, ethoxycarbonyl, propioxycarbonyl, trityl and trichloroethyl, and in the products of formulae (IVA) and (IVB) the substituent $R_1p$ represents the substituent $R_1$, as defined in claim 1, the optionally present hydroxy or amino groups of which are optionally protected as mentioned above, it being understood that in the products of formulae (VA) and (VB) $R_1p$ does not represent the same values as $R_1$.